# EUROPEAN PATENT APPLICATION

(11) **EP 1 795 189 A2**
(43) Date of publication of application: **13.06.2007**
(21) Application number: 07007006.5
(22) Date of filing: 20.03.2003
(51) Int. Cl.: A61K 9/70, A61K 31/485, A61P 25/24

(54) **Method of administering buprenorphine to treat depression**

(30) Priority: 20.03.2002 US 366358 P
(62) Divisional of application: 03721427.7
(71) Applicant: EURO-CELTIQUE S.A., 2330 Luxembourg (LU)
(72) Inventor: Kaiko, Robert F., Weston CT 06883 (US); Sanchez, Ramiro, Hamden CT 06518 (US)
(74) Representative: Maiwald Patentanwalts GmbH

(57) **Abstract**

A method of treating depression using transdermal delivery of buprenorphine is described. In one embodiment, the method employs transdermal patches comprising buprenorphine, preferably escalating incrementally the buprenorphine dose to a level where one or more symptoms of depression are alleviated. The method is particularly suitable for r patients suffering from refractory depression, or for patients suffering from both depression and pain.

## Description

This application claims priority from U.S. Serial No. 60/366,358, filed March 20, 2002, which is hereby incorporated by reference in its entirety.

### FIELD OF THE INVENTION

The present invention contemplates a method of alleviating the symptoms of depression, particularly refractory depression, by administration of a buprenorphine in a transdermal dosage form.

### BACKGROUND OF THE INVENTION

It is estimated that as many as one in ten Americans will suffer from depression at some point during their lifetime. The term "depression" covers a wide range of illnesses, from mild to moderate to severe, and even life-threatening forms. However, they all share common psychological, behavioral, cognitive, physical, and emotional manifestations. Differences between depression subtypes, such as those articulated in the Diagnostic and Statistical Manual of Mental Disorders, (4th Ed., Washington, DC, American Psychiatric Association, 2000), depend upon the range of severity, frequency, and duration of these defining attributes.

Symptoms of depression include, but are not limited to, a persistent sad mood, loss of interest or pleasure in activities that were once enjoyed, significant change in appetite or body weight, difficulty sleeping or oversleeping, physical slowing or agitation, loss of energy, feelings of worthlessness or inappropriate guilt, difficulty thinking or concentrating, and recurrent thoughts of death or suicide.

Breakthrough therapies using selective serotonin reuptake inhibitors (SSRIs, e.g., sertraline, olanzapine, and fluoxetine) have helped a large proportion of those suffering from depression. While approximately eighty percent of people with depression respond very positively to treatment (e.g., pharmacological intervention), some respond only partially to treatment with SSRIs or the older tricyclics, and a significant number of individuals remain treatment refractory to these therapeutic approaches. For severe refractory depression, electroconvulsive treatment (ECT) has been used.

Opium has been used in treatment of various psychiatric disorders, possibly since medieval times (Weber et al., Int Clin Psychopharmacology 1988;3:255-266). Emrich (In: Typical and Atypical Antidepressants: Clinical Practice, Costa et al. (Ed.), Raven Press, New York (1982), p. 77 *et seq.)* has suggested that because of the euphoric, tranquilizing, and anti-anxiety actions of opioids, a functional deficiency of endogenous opioids may underlie the pathogenesis of endogenous depression (see Extein et al., Am J Psychiatry 1980;137:845-846). While administration of an endogenous opioid (β-endorphin) to depressed patients in some studies have provided no or little observable therapeutic effect (Gold et al., Am J Psychiatry 1979;136:982-983), researchers have tested whether synthetic opioid drugs could be helpful in treating depression. For example, Shapira et al. (J Clin Psychiatry 2001;62:205-206) found that tramadol helped a patient with refractory major depression, and Lehmann et al. (Curr Therapeutic Res 1971;13:42-49) tested a combination therapy of meperidine hydrochloride (demerol) and dextroamphetamine (dexedrine), finding that some patients were helped, but the treatment regimen had differential effects on various depressive symptoms.

On the other hand, it has been reported that codeine showed no anti-depressive effect (Varga et al., Ann NY Acad Sci 1982;398:103-105), and for morphine and methadone, the results are conflicting (Extein, Psychopharmacology Bull 1981;1:29-33; Feinberg et al., Research Monograph Series 43, National Institute on Drug Abuse, 1982; Proceedings of the 44th Annual Scientific Meeting, 1982; 245-250; Abse et al., Ann NY Acad Sci 1982;398:79-83; Goldstein, Biol Psychiatry 1984; 19:1272-3).

Buprenorphine is also among the opioids suggested for use in anti-depressive therapy (Paetzold et al., Nervenheilkunde 2000;19:143-150; Callaway Soc Biol Psychiatry 1996;39:989-990; Emrich et al., Neuropharmacology 1983;22:385-388). For example, in a 7-patient study where subjects suffering from refractory depression received multiple daily doses of buprenorphine sublingually or intranasally, some patients achieved complete remission (Bodkin et al., J Clin Psychopharmacology 1995;15:49-57). In another study, patients suffering from major depressive disorder received two daily doses of sublingual buprenorphine, resulting in significant improvement of the patients' status (Emrich et al., Ann NY Acad Sci 1982;398:108-112, and Lancet 1982;2:709). The same study reported, however, that most of the patients experienced some degree of slight nausea, dizziness, and sedation, and one patient vomited.

Despite advances in the art, there remains a need for methods of effectively treating patients suffering from depression, especially refractory depression. These concerns are particularly acute with respect to providing a safe and effective method of management of the disorder.

### SUMMARY OF THE INVENTION

The present invention contemplates a method of alleviating one or more symptoms of depression, the method comprising administering to the patient a transdermal dosage form comprising buprenorphine.

Accordingly, the invention provides for a method of treating depression, which method comprises administering a transdermal dosage form comprising buprenorphine to a patient suffering from depression, thereby alleviating one or more symptoms of depression in the patient. The transdermal dosage form is preferably a transdermal dosage article or a transdermal dosage composition. For example, the transdermal dosage article may be a diffusion driven transdermal system, and the transdermal dosage composition can be selected from a topical gel, a lotion, an ointment, a transmucosal system, a transmucosal device, and an iontophoretic delivery system. In one embodiment, the transdermal dosage form comprises from about 5 to about 40 mg buprenorphine. Symptoms of depression may include, but are not limited to, persistent sad mood, loss of interest or pleasure in activities that were once enjoyed, significant change in appetite or body weight, difficulty sleeping, oversleeping, physical slowing, agitation, loss of energy, feelings of worthlessness, inappropriate guilt, difficulty thinking, difficulty concentrating, and recurrent thoughts of death or suicide. In a particular embodiment, the depression is classified as refractory depression. In yet another embodiment, the patient is an elderly patient. In still another embodiment, the method comprises repeating the step of administering the transdermal dosage form at least once, preferably at least 6 times. The repeating step may be conducted, for example, every 3-7 days.

The invention also provides a method of treating depression, which method comprises sequential administration of a first, a second, and a third transdermal dosage article comprising buprenorphine to a patient suffering from depression, wherein the third dosage article comprises a higher dosage of buprenorphine than the first and second dosage article thereby alleviating one or more symptoms of depression in the patient. In one embodiment, the first dosage article comprises no more than 5 mg buprenorphine, the second dosage article comprises no more than 10 mg buprenorphine and is administered for a dosing period of three days, and the third dosage article comprises at least 20 mg buprenorphine and is administered for a dosing period of at least 2 days. In another embodiment, the first dosage article comprises no more than 10 mg buprenorphine, the second dosage article comprises no more than 20 mg buprenorphine and is administered for three days, and the third dosage article comprises at least 30 mg buprenorphine and is administered for at least 2 days. In yet another embodiment, the first dosage article comprises no more than 20 mg buprenorphine, the second dosage article comprises no more than 30 mg buprenorphine and is administered for three days, and the third dosage article comprises 40 mg buprenorphine and is administered for at least 2 days. The second dosage article may be administered, for example, between 3 and 7 days after the first dosage article, and the third dosage article may be administered, for example, between 3 and 7 days after the second dosage article. In a preferred embodiment, the administration of the third dosage article is repeated at least once. In another preferred embodiment, the administration of the third dosage article is repeated at least 4 times. The patient can be, for example, an elderly patient.

### DETAILED DESCRIPTION OF THE INVENTION

The present invention discloses a method of transdermally administering buprenorphine to alleviate the symptoms of depression in a patient. In a preferred embodiment, buprenorphine is administered in a transdermal dosage form.

In patients suffering from depression, transdermal delivery of buprenorphine offers several advantages. Patients with symptoms of depression have received frequent administrations of buprenorphine by oral or sublingual routes. However, such regimens rely heavily on patient compliance, requiring a depressed patient to diligently maintain the dosage schedule. In addition, depression may last for extended periods of time, and sometimes is more or less a chronic disease. Transdermal administration of buprenorphine provides for delivery of the drug for many days, using a slow release dosage form that minimizes the problems of frequent administration. The method also increases the degree of patient compliance with drug therapy and treatment efficacy. Notably, the reduction in side effects and minimization of complications does not diminish the primary therapeutic effect: treatment of depression.

The treatment schedule of the invention may comprise administering a buprenorphine transdermal form for a predefined number of days, e.g., between 1-10 days, preferably for 3-7 days. Thereafter, another buprenorphine transdermal dosage form, containing the same or different dose of BTDS, may be administered for another period of time, and so on. Preferred dosage levels of buprenorphine for treatment of depression are those containing 2-100 mg of buprenorphine, preferably 5-40 mg. For example, a transdermal patch may contain 5, 10, 20, 30, or 40 mg buprenorphine in suitable formulation.

As used herein, the term "predefined number of days" refers to the length of time during which a transdermal buprenorphine dosage form is administered to the patient. The predefined number of days may vary between individuals and may be determined by one of ordinary skill in the art using the guidelines discussed within the present application. In a preferred embodiment, the predefined number of days is 3-7 days.

The dosage regimen of the present invention may alternatively be described in terms of administration of a "series of transdermal dosage forms comprising at least one incremental dosage of buprenorphine." This refers to the sequential administration of at least two transdermal dosage forms to the patient, wherein the dosage of buprenorphine in the first dosage form is less than at least one subsequently administered dosage form, each subsequently administered dosage form being administered a predefined number of days, e.g., 1-10 days, preferably 3-7 days, after the prior dosage form. For example, a series of three transdermal dosage forms may be administered in the dosage regimen, wherein the first dosage form contains 5 mg buprenorphine, the second dosage form contains 10 mg buprenorphine, and the third dosage form contains 20 mg buprenorphine, such that each subsequent dosage form in the series has twice the dosage of buprenorphine than its predecessor. Alternatively, the series of dosage forms may include 20 mg, 30 mg, and 40 mg buprenorphine respectively, or 2 mg, 4 mg, and 8 mg buprenorphine, respectively, or 1 mg, 2 mg, or 3 mg buprenorphine, respectively. For a series of three patches, particular dosage regimens (in mg) can be 5-5-10, 5-10-10, 5-10-20, 5-20-40, 5-10-30, 5-30-40, 10-10-20, 10-10-30, 10-10-40, 10-20-30, 10-20-40, and 10-30-40. Once a dosage level has been reached that alleviates one or more symptoms of depression, the patient can be maintained at the same dosage level for as long as is needed to treat the depression.

As used herein, "BTDS" means "Buprenorphine Transdermal System", and "BTDS X", wherein "X" is a number higher than zero, means a transdermal dosage form containing X milligrams of buprenorphine. Thus, "BTDS 5" contains about 5 mg buprenorphine. Preferably, a BTDS contains buprenorphine in the form of a base or a salt, more preferably in the form of a base.

In a preferred embodiment, the transdermal dosage form is selected from the group consisting of a transdermal dosage article and transdermal dosage composition. The transdermal dosage article may be a diffusion-driven transdermal system, and the transdermal dosage composition may be selected from the group consisting of topical gel, lotion, ointment, transmucosal system, transmucosal device, and iontohoretic delivery system. In one embodiment, the transdermal dosage form comprises from about 0.01% to about 90% by weight of buprenorphine based upon 100 % total weight of the dosage. In a preferred embodiment, transdermal preparations contain from about 0.5% to about 25% by weight of the compound, salt or derivative, and more preferably from about 0.5% to about 10% by weight of buprenorphine. In an alternative embodiment, the transdermal dosage form of the invention provides buprenorphine at a delivery rate of about 1 µg/hr to about 500 µg/hr, preferably about 5 µg/hr to about 200 µg/hr, about 10 µg/hr to about 100 µg/hr, or about 40 µg/hr to about 60 µg/hr.

In one embodiment, treatment with buprenorphine may be used in combination with other therapeutic methods, such as the administration of SSRIs, tricyclics, monoamine oxidase inhibitors, and combinations thereof. Thus, the method of the present invention contemplates the simultaneous administration of buprenorphine and other anti-depressants in a single dosage form, as well as co-administration of buprenorphine and other anti-depressants, e.g., by a single or multiple dosage form. Alternatively, additional anti-depressants may be administered by any acceptable method, e.g., parenteral administration.

The method of the present invention may be used for any patient in need of treatment for depression or symptoms of depression, including elderly patients (age over 65 years), young adult patients (age between 17 and 45 years), and pediatric patients (age between birth and 16 years, including age groups often referred to as neonates, infants, children, and adolescent).

As used herein, the term "depression" refers to a mental state of depressed mood characterized by feelings of sadness, despair and discouragement. Depression ranges from normal feelings of "the blues" through dysthymia to major depression. It is often marked by a persistent sad mood, loss of interest or pleasure in activities that were once enjoyed, significant change in appetite or body weight, difficulty sleeping or oversleeping, physical slowing or agitation, loss of energy, feelings of worthlessness or inappropriate guilt, difficulty thinking or concentrating, and recurrent thoughts of death or suicide. The symptoms of depression may vary from mild to moderate to severe. Differences between depression subtypes depend upon the range of severity, frequency, and duration of these defining attributes. Depression is generally categorized as major depression, bipolar I disorder, bipolar II disorder, dysthymic disorder, and cyclothymic disorder. Definitions of the criteria for classification and the symptoms of depression (listed above) can be determined from the DSM-IV (Diagnostic and Statistical Manual of Mental Disorders (4th ed.), American Psychiatric Association).

Depression may be assessed using several self-surveys and physician-completed assessments. Self-surveys include, but are not limited to, the Zung Self-Rating Depression Scale The physician completed assessments include, but are not limited to, the Beck Depression Inventory, Depression Screening with SALSA, Depression Screening with SIG E CAPS, and the Hamilton Rating Scale for Depression. In a preferred embodiment, depression is assessed using the Structured Clinical Interview for Diagnosis, i.e., SCID. The surveys and assessments are well known to those of ordinary skill in the art and can be used as directed. As used herein, the term "alleviate" or "alleviating" refers to at least partially mitigating or attenuating one or more symptoms of depression. Such alleviation can be assessed by a physician or by the patient using one or more of the self-surveys described herein.

In a specific embodiment, the method of the invention is used for treating patients suffering from a depression subtype termed "refractory depression". Patients with refractory depression have previously not responded or only partially responded to standard medications such as, but not limited to, selective serotonin reuptake inhibitors *(e.g.,* fluoxetine, paroxetine, and sertraline); tricyclic antidepressants *(e.g.,* amitriptyline, desipramine, imipramine, and nortriptyline); and monoamine oxidase inhibitors *(e.g.,* phenelzine and tranylcypromine). A partial response is characterized by an improvement of less than 50% on the HAMD-21 or Montgomery-Asberg Depression Rating Scale, preferably from about 1% to about 49%, more preferably from about 10 % to about 49%, most preferably from about 15 % to about 49%. In one embodiment, patients suffering from refractory depression are treated by a combination therapy, combining transdermal buprenorphine with administration of one or more other anti-depressant agents, *e.g.,* SSRIs, tricyclics, monoamine oxidase inhibitors, or with treatments such as electroconvulsive therapy.

In one embodiment, the patient suffering from depression is further in need of pain treatment. For example, the patient may be classified as having a specific medical condition associated with pain. Such conditions are well known in the art and include, for example, cancer. The patient may, if needed, be on additional medication to control pain. Such medications include, but are not limited to, non-steroidal anti-inflammatory drugs (NSAIDS), acetaminophen (or paracetamol) and immediate-release mu-agonist opioids, and combinations thereof. The present invention may also be used to supplant existing medications for pain or depression, thereby reducing the need for other types of medication.

The term "adverse event" or "adverse experience" herein means any untoward medical occurrence in a patient or clinical investigation subject administered a pharmaceutical product. Exemplary adverse events in a treatment regimen include, but are not limited to, nausea, constipation, vomiting, headache, dizziness, somnolence, orthostatic hypotension, respiratory depression, choleocystitis, and abdominal pain.

Certain preferred embodiments of the present invention are described below. Insofar as the description refers to certain components of the invention with approximations, e.g., the terms "about" or "approximately", these terms shall generally mean an acceptable degree of error for the quantity measured given the nature or precision of the measurements. Typical, exemplary degrees of error are within 20 percent (%), preferably within 10%, and more preferably within 5% of a given value or range of values. Numerical quantities given herein are approximate unless stated otherwise.

### Buprenorphine

The present invention relates to the use of buprenorphine or a pharmaceutically acceptable salt, ether derivative, ester derivative, acid derivative, enantiomer, diasteriomer, racemate, polymorph, or solvate thereof, that has anti-depressant activity. This molecule has the chemical formula N-cyclopropylmethyl-7α-((S)-1-hydroxy- 1,2,2-trimethylpropyl)-6,14-endo-ethano-6,7,8,14-tetrahydronororipavine.

Buprenorphine has been shown to be effective to control pain in a wide range of patients when delivered by a number of different routes of administration, including intravenously, epidurally, intrathecally, or sublingually in both young and elderly patients (Inagaki et al., Anesth Analg 1996, 83:530-536; Brema et al., Int J Clin Pharmacol Res 1996, 16:109-116; Capogna et al., Anaesthesia 1988, 43:128-130; Adrianensen et al., Acta Anaesthesiol Belg 1985, 36:33-40; Tauzin-Fin et al., Eur J Anaesthesiol 1998, 15:147-152; Nasar et al., Curr Med Res Opin 1986, 10:251-255). There are several types of transdermal formulations of buprenorphine reported in the literature. See, for example, U.S. Pat. No. 5,240,711 to Hille et al., U.S. Pat. No. 5,225,199 to Hidaka et al., U.S. Pat. No. 5,069,909 to Sharma et al., U.S. Pat. No. 4,806,341 to Chien et al.; U.S. Pat. No. 5,026,556 to Drust et al.; U.S. Pat. No. 5,613,958 to Kochinke et al.; and U.S. Patent No. 5,968,547 to Reder et al. Transdermal delivery systems of buprenorphine, made by Lohmann Therapie-Systeme GmbH & Co., are currently sold in the European Union under the trademark name TRANSTEC^{®}. These patches contain 20, 30, or 40 mg of buprenorphine, with an approximate delivery or "flux" rate of 35, 52.5, and 70 µg/hr, respectively.

Pharmacologically, buprenorphine is a partial agonist against the µ-opioid receptor, possessing both agonist and antagonist properties. A partial agonist is an agent that binds to, but does not fully stimulate, the receptor. In addition, the agent prevents the binding of a full agent, thereby blocking the total pharmacologic activity possible from the receptor. Partial agonists exhibit ceiling effects (i.e., increasing the dose only has effects up to a certain level). Therefore, partial agonists have greater safety indices than full agonists (such as heroin or morphine and certain analgesic products chemically related to morphine).

The present invention also contemplates the use of other partial agonists, such as N-but-3-enyl-norbuprenorphine or a pharmaceutically acceptable salt thereof. This composition is described in PCT publication WO 02/070524, which is incorporated herein by reference in its entirety.

In the context of the present invention, the term "effective amount" is that amount of buprenorphine or salt thereof that is sufficient to reduce or relieve symptoms of depression in a patient. In so far as the present invention also contemplates the administration of a buprenorphine to treat pain as well as depression, buprenorphine will be administered in an "analgesic effective amount", i.e., an amount of buprenorphine that reduces or alleviates pain in the patient, as determined by the degree of pain suffered by the patient, together with such factors as the height, weight, age, and condition of the patient, as well as the particular transdermal dosage form. In this regard, pain can be categorized by a Visual Analog Scale (VAS).

The use of various pharmaceutically acceptable salts, ether derivatives, ester derivatives, acid derivatives, and aqueous solubility altering derivatives of the buprenorphine also are encompassed by the present invention. The present invention further includes all individual enantiomers, diastereomers, racemates, and other isomer ratios of the compound. The invention also includes the use of all buprenorphine polymorphs and solvates, such as hydrates and those formed with organic solvents, which have an anti-depressant effect. Such isomers, polymorphs, and solvates may be prepared by methods known in the art, such as by regiospecific and/or enantioselective synthesis and resolution, based on the disclosure provided herein.

### Salts and Derivatives

The use of various pharmaceutically acceptable salts, and active ether derivatives, ester derivatives, acid derivatives, and aqueous solubility altering derivatives of the active compound also are encompassed by the present invention. The present invention further includes the use of all individual active enantiomers, diastereomers, racemates, and other isomers of the compound, as well as combinations thereof. The invention also includes all polymorphs and solvates, such as hydrates and those formed with organic solvents, of this compound. Such isomers, polymorphs, and solvates may be prepared by methods known in the art, such as by regiospecific and/or enantioselective synthesis and resolution, based on the disclosure provided herein.

Suitable salts of the compound include, but are not limited to, acid addition salts, such as those made with hydrochloric, hydrobromic, hydroiodic, perchloric, sulfuric, nitric, phosphoric, acetic, propionic, glycolic, lactic pyruvic, malonic, succinic, maleic, fumaric, malic, tartaric, citric, benzoic, carbonic cinnamic, mandelic, methanesulfonic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid; salts made with saccharin; alkali metal salts, such as sodium and potassium salts; alkaline earth metal salts, such as calcium and magnesium salts; and salts formed with organic or inorganic ligands, such as quaternary ammonium salts.

Additional suitable salts include, but are not limited to, acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate salts of buprenorphine.

The present invention includes prodrugs of the compound of the present invention. Prodrugs include, but are not limited to, functional derivatives of buprenorphine that are readily convertible in vivo into buprenorphine. Conventional procedures for the selection and preparation of suitable prodrug derivatives are described, for example, in "Design of Prodrugs", ed. H. Bundgaard, Elsevier, 1985.

### Pharmaceutical Compositions

The compound(s) of the present invention may be formulated into a pharmaceutical composition. The pharmaceutical composition also may include additives, such as a pharmaceutically acceptable carrier, a preservative, a dye, a binder, a suspending agent, a dispersing agent, a colorant, a disintegrant, an excipient, a diluent, a lubricant, a plasticizer, or any combination of any of the foregoing. In addition, the composition may be formulated with an antagonist or inactivating agent to prevent misuse of the active agent.

Suitable pharmaceutically acceptable carriers include, but are not limited to, ethanol, water, glycerol, aloe vera gel, allantoin, glycerin, vitamin A and E oils, mineral oil, PPG2 myristyl propionate, vegetable oils and solketal. The composition may also include suitable preservatives, e.g., sodium benzoate, and other additives that may render the composition more suitable for transdermal use. Suitable dispersing and suspending agents include, but are not limited to, synthetic and natural gums, such as vegetable gum, tragacanth, acacia, alginate, dextran, sodium carboxymethylcellulose, methylcellulose, polyvinyl-pyrrolidone and gelatin. Suitable pharmaceutical diluents include, but are not limited to, water. Examples of additional additives include, but are not limited to, sorbitols; talcs; stearic acids; and dicalcium phosphate.

### Unit Dosage Forms

Solid unit dosage forms may be prepared by mixing the compound, salt or derivative of the present invention with a pharmaceutically acceptable carrier and any other desired additives as described above. The mixture is typically mixed until a homogeneous mixture of the compound of the present invention and the carrier and any other desired additives are formed, i.e., until the compound is dispersed evenly throughout the composition.

Biodegradable polymers for controlling the release of the compound also may be included in the composition. These polymers include, but are not limited to, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.

### Transdermal Dosage Forms

Transdermal dosage forms are convenient dosage forms for delivering many different active therapeutically effective agents, including but not limited to analgesics, such as for example, opioid analgesics. Typical opioid analgesics include, but are not limited to, fentanyl, buprenorphine, etorphines, and other high potency narcotics. Transdermal dosage forms are particularly useful for timed release and sustained release of active agents.

Transdermal dosage forms may be classified into transdermal dosage articles and transdermal dosage compositions. The most common transdermal dosage article is a diffusion driven transdermal system (transdermal patch) using either a fluid reservoir or a drug in adhesive matrix system. Transdermal dosage compositions include, but are not limited to, topical gels, lotions, ointments, transmucosal systems and devices, and iontophoretic (electrical diffusion) delivery systems. Preferably, the transdermal dosage form is a transdermal patch.

The pharmaceutical compositions can be formulated as transdermal dosage forms, such as a diffusion driven transdermal system (transdermal patch) using either a fluid reservoir or a drug in an adhesive matrix system, a topical gel, a lotion, an ointment, a transmucosal system, a transdermal patch, and an iontophoretic (electrical diffusion) delivery system. The transdermal dosage form is used in the dosage regimen of the present invention for timed release or sustained release of buprenorphine.

Transdermal dosage forms used in accordance with the invention preferably include a backing layer made of pharmaceutically acceptable material which is impermeable to buprenorphine. The backing layer preferably serves as a protective cover for buprenorphine and may also provide a support function. Examples of materials suitable for making the backing layer are films of high and low density polyethylene, polypropylene, polyvinylchloride, polyurethane, polyesters such as poly(ethylene phthalate), metal foils, metal foil laminates of such suitable polymer films, textile fabrics, if the components of the reservoir cannot penetrate the fabric due to their physical properties and the like. Preferably, the materials used for the backing layer are laminates of such polymer films with a metal foil such as aluminum foil. The backing layer can be any appropriate thickness which will provide the desired protective and support functions. A suitable thickness will be from about 10 to about 200 microns. Desirable materials and thickness will be apparent to the skilled artisan.

In certain preferred embodiments, the transdermal dosage forms used in accordance with the invention contain a polymer matrix layer. Generally, the polymers used to form the pharmaceutically acceptable polymer matrix are those capable of forming thin walls or coatings through which pharmaceuticals can pass at a controlled rate. A non-limiting list of exemplary materials for inclusion in the polymer matrix includes polyethylene, polypropylene, ethylene/propylene copolymers, ethylene/ethyl-acrylate copolymers, ethylenevinyl acetate copolymers, silicones, rubber, rubber-like synthetic homo-, co- or block polymers, polyacrylic esters and the copolymers thereof, polyurethanes, polyisobutylene, chlorinated polyethylene, polyvinylchloride, vinyl chloride-vinyl acetate copolymer, polymethacrylate polymer (hydrogel), polyvinylidene chloride, poly(ethylene terephthalate), ethylene-vinyl alcohol copolymer, ethylene-vinyloxyethanol copolymer, silicones including silicone copolymers such as polysiloxane-polymethacrylate copolymers, cellulose polymers (e.g., ethyl cellulose, and cellulose esters), polycarbonates, polytetrafluoroethylene and mixtures thereof. Exemplary materials for inclusion in the polymer matrix layer are silicone elastomers of the general polydimethylsiloxane structures, (e.g., silicone polymers). Preferred silicone polymers cross-link and are pharmaceutically acceptable. Other preferred materials for inclusion in the polymer matrix layer include: silicone polymers that are cross-linkable copolymers having dimethyl and/or dimethylvinyl siloxane units which can be crosslinked using a suitable peroxide catalyst. Also preferred are those polymers consisting of block copolymers based on styrene and 1,3-dienes (particularly linear styrene-isoprene-block copolymers of styrene-butadiene-block copolymers), polyisobutylenes, polymers based on acrylate and/or methacrylate.

The polymer matrix layer may optionally include a pharmaceutically acceptable crosslinking agent. Suitable crosslinking agents include, e.g., tetrapropoxy silane. Preferred transdermal delivery systems used in accordance with the methods of the present invention include an adhesive layer to affix the dosage form to the skin of the patient for a desired period of administration, e.g., about 2 to about 8 days. If the adhesive layer of the dosage form fails to provide adhesion for the desired period of time, it is possible to maintain contact between the dosage form with the skin by, for instance, affixing the dosage form and the skin of the patient with an adhesive tape, e.g., surgical tape. Adhesion of the dosage form to the skin of the patient can be achieved solely by the adhesive layer of the dosage form or in connection with a peripheral adhesive source, such as surgical tape, but the dosage form should preferably be adhered to the patient's skin for the requisite administration period.

The transdermal patch may include a skin-facing layer containing the active agent and having a skin-facing side and a top-facing side and an optional opposing top-facing layer containing an optional antagonist and/or inactivating agent, which has a skin-facing side and an opposing top-facing side. These layers may optionally be contained on opposite sides of a single membrane having a skin-facing side and an opposing top-facing side, or may be separated by more than one membrane stacked on one another, the skin-facing side of the outer layer facing the top-facing layer of the inner layer. An optional layer, which could be impermeable or selectively permeable, similarly having a skin-facing side and a top-facing side, may separate, completely or partially, the active agent-containing layer from the inactive agent-containing layer An optional additional adhesive layer and/or a release layer, each also having respective skin-facing and top-facing sides, may cover all or part of the layer containing the active agent. An optional cover layer having skin-facing and a top-facing sides may cover all or part of the top-facing side or the skin-facing side of the layer containing the inactivating agent.

The adhesive layer preferably includes using any adhesive known in the art that is pharmaceutically compatible with the dosage form and preferably hypoallergenic, such as polyacrylic adhesive polymers, acrylate copolymers (e.g., polyacrylate) and polyisobutylene adhesive polymers. In other preferred embodiments of the invention, the adhesive is a pressure-sensitive contact adhesive, which is preferably hypoallergenic. The transdermal dosage forms which can be used in accordance with the present invention may optionally include a permeation enhancing agent. Permeation enhancing agents are compounds which promote penetration and/or absorption of the buprenorphine into the blood stream of the patient. A non-limiting list of permeation enhancing agents includes polyethylene glycols, surfactants, and the like.

Alternatively, permeation of buprenorphine may be enhanced by occlusion of the dosage form after application to the desired site on the patient with, e.g. an occlusive bandage. Permeation may also be enhanced by removing hair from the application site by, e.g., clipping, shaving or use of a depilatory agent. Another permeation enhancer is heat. It is thought that heat enhancement can be induced by, among other things, using a radiating heat form, such as an infrared lamp, onto the application site after application of the transdermal dosage form. Other means of enhancing permeation of buprenorphine such as the use of iontophoretic means are also contemplated to be within the scope of the present invention.

A preferred transdermal dosage form which may be used in accordance with the present invention includes a non-permeable backing layer made, for example, of polyester; an adhesive layer made, for example of a polyacrylate; and a matrix containing the buprenorphine and other desirable pharmaceutical aids such as softeners, permeability enhancers, viscosity agents and the like. The active agent, buprenorphine, may be included in the device in a drug reservoir, drug matrix or drug/adhesive layer. This area of the patch, and the amount of active per unit area determine the limit dose, as one of ordinary skill in the art can readily determine.

Certain preferred transdermal delivery systems also include a softening agent. Suitable softening agents include higher alcohols such as dodecanol, undecanol, octanol, esters of carboxylic acids, wherein the alcohol component may also be a polyethoxylated alcohol, diesters of dicarboxylic acids, such as di-n-butyladiapate, and triglycerides particularly medium-chain triglycerides of the caprylic/capric acids or coconut oil, have proved to be particularly suitable. Further examples of suitable softeners are multivalent alcohols, for example, levulinic acid, cocprylic acids glycerol and 1,2-propanediol which can also be etherified by polyethylene glycols.

A buprenorphine solvent may also be included in the transdermal delivery systems of the present invention. Preferably, the solvents dissolve the buprenorphine to a sufficient extent thereby avoiding complete salt formation. A non-limiting list of suitable solvents include those with at least one acidic group. Particularly suitable are monoesters of dicarboxylic acids such as monomethylglutarate and monomethyladipate. Other pharmaceutically acceptable compounds which may be included in the reservoir or matrix include: solvents, for example alcohols such as isopropanol; permeation enhancing agents such as those described above; and viscosity agents, such as cellulose derivatives, natural or synthetic gums, such as guar gum, and the like.

In preferred embodiments, the transdermal dosage form includes a removable protective layer. The removable protective layer is removed prior to application, and may consist of the material used for the production of the backing layer described above provided that it is rendered removable, for example, by a silicone treatment. Other removable protective layers, for example, are polytetrafluoroethylene, treated paper, allophane, polyvinyl chloride, and the like. Generally, the removable protective layer is in contact with the adhesive layer and provides a convenient means of maintaining the integrity of the adhesive layer until the desired time of application.

The composition of the transdermal dosage forms used in accordance with the invention and the type of device used are not considered critical to the method of the invention, provided that the device delivers the active agent, e.g. buprenorphine, for the desired time period and at the desired flux rate and/or the desired delivery rate of the transdermal dosage form.

Certain preferred transdermal dosage forms for use in accordance with the present invention are described in U.S. Pat. No. 5,240,711 to Hille, et. al.; (assigned to LTS Lohmann Therapie-Systeme GmbH & Co.), hereby incorporated by reference. Such buprenorphine transdermal delivery systems may be a laminated composite having an impermeable backing layer containing buprenorphine, and optionally, a permeation enhancer combined with a pressure-sensitive adhesive. A preferred transdermal dosage form in accordance with U.S. Patent No. 5,240,711 includes: (i) a polyester backing layer which is impermeable to buprenorphine; (ii) a polyacrylate adhesive layer; (iii) a separating polyester layer; and (iv) a matrix containing buprenorphine, a solvent for the buprenorphine, a softener and a polyacrylate adhesive. The buprenorphine solvent may or may not be present in the final formulation. The transdermal delivery device described therein includes a backing layer which is impermeable to the active substance, a pressure-sensitive adhesive reservoir layer and optionally, a removable protective layer. Preferably, the reservoir layer includes about 10 to about 95 % (by weight) polymeric material, about 0.1 to about 40% (by weight) softener, about 0.1 to about 30% (by weight) buprenorphine. A solvent for the buprenorphine base or pharmaceutically acceptable salt thereof may be included as about 0.1 to about 30 % (by weight).

The dosage forms of the present invention may also include one or more inactivating agents. The term "inactivating agent" refers to a compound that inactivates or crosslinks the active agent, in order to decrease the abuse potential of the transdermal dosage form. Non limiting examples of inactivating agents include, but are not limited to, polymerizing agents, photinitiators, and formalin. Examples of polymerizing agents include diisocyanates, peroxides, diimides, diols, triols, epoxides, cyanoacrylates, and UV activated monomers.

Generally, topical preparations contain from about 0.01 to about 100% by weight and preferably from about 3 to about 80% by weight of buprenorphine, based upon 100% total weight of the topical preparation. Generally, transdermal dosage forms contain from about 0.01 to about 100% by weight and preferably from about 3 to about 50% by weight of the compound, based upon 100% total weight of the dosage.

The method of the present invention preferably administers buprenorphine in a way that achieves a gradual increase in the plasma concentration of buprenorphine in the patient. In a preferred embodiment, the plasma profile achieved by the method of the present invention may be described as follows: (a) the mean plasma buprenorphine concentration 24 hours after administration is between 10-100 pg/ml, preferably 20-50 pg/ml; (b) the mean plasma buprenorphine concentration 72 hours after administration is between 25-200 pg/ml, preferably 40-100 pg/ml; (c) the mean plasma buprenorphine concentration 144 hours after administration is between 100-250 pg/ml, preferably 150-200 pg/ml; and (d) the mean plasma buprenorphine concentration 168 hours after administration is between 400-1000 pg/ml, preferably at least 500 pg/ml, or higher depending on the patient's need.

The present invention also provides a method of treating depression in a human patient, comprising administering buprenorphine transdermally to the patient by applying a transdermal delivery system to the skin of a patient, and maintaining the transdermal delivery system in contact with the patient's skin for at least 3 days, the transdermal delivery system maintaining a mean relative release rate of from about 3 µg/hr to about 200 µg/hr, such that the following mean plasma concentrations are achieved: (a) a mean plasma concentration from about 0.3 to about 200 pg/ml at about 6 hours after initiation of the dosing interval; (b) a mean plasma concentration from about 3 to about 400 pg/ml at about 12 hours after initiation of the dosing interval; (c) a mean plasma concentration from about 7 to about 1000 pg/ml at about 24 hours after initiation of the dosing interval; (d) a mean plasma concentration from about 13 to about 1200 pg/ml at about 36 hours after initiation of the dosing interval; (e) a mean plasma concentration from about 16 to about 1500 pg/ml at about 48 hours after initiation of the dosing interval; (f) a mean plasma concentration from about 20 to about 1500 pg/ml at about 60 hours after initiation of the dosing interval; (g) a mean plasma concentration from about 21 to about 1600 pg/ml at about 72 hours after initiation of the dosing interval; and (h) a mean plasma concentration from about 19 to about 1600 pg/ml over at least the next 48 hours.

The invention also provides for maintaining the buprenorphine transdermal delivery system in contact with the patient's skin such that the mean plasma concentrations are maintained as follows: (a) a mean plasma concentration from about 23 to about 1500 pg/ml at about 96 hours after initiation of the dosing interval; (b) a mean plasma concentration from about 23 to about 1500 pg/ml at about 120 hours after initiation of the dosing interval; (c) a mean plasma concentration from about 22 to about 1400pg/ml at about 144 hours after initiation of the dosing interval; and a mean plasma concentration from about 19 to about 1200 pg/ml at about 168 hours after initiation of the dosing interval.

Topical preparations typically contain a suspending agent and optionally, an antifoaming agent. Such topical preparations may be liquid drenches, alcoholic solutions, topical cleansers, cleansing creams, skin gels, skin lotions, and shampoos in cream or gel formulations (including, but not limited to aqueous solutions and suspensions).

The compound of the present invention also can be administered in the form of liposome delivery systems, such as small unilamellar vesicles, large unilamellar vesicles and multilamellar vesicles that may be included in the transdermal article or transdermal composition. Liposomes can be formed from a variety of phospholipids, such as cholesterol, stearylamine or phosphatidylcholines.

The transdermal dosage form may be formulated by any method known in the art and may be administered as suggested. Such formulations are described in U.S. Patents 4,806,341; 5,240,711; and 5,968,547.

### Administration

The unit dosage forms of the present invention are administered to a patient suffering from depression, optionally also from pain. In one embodiment, the patient is elderly. The unit dosage forms of the present invention may be administered at the defined dosing regimen comprising several discrete dosing periods in order to obtain optimal activity while minimizing any potential toxicity. A dosing period is the time during which one of the transdermal dosage forms in the series is administered to the patient, and the dosing regimen will consist of a separate dosing period for administration of each transdermal dosage form in the series.

In one embodiment, the method involves administering to the patient an analgesic effective amount of buprenorphine in a dosage regimen comprising administering to the patient a series of transdermal dosage forms comprising graduated and ascending dosages of buprenorphine. Preferably, the dosage regimen comprises the steps of: (a) administering to the patient a first buprenorphine-containing transdermal dosage form for a first dosing period; (b) administering to the patient a second buprenorphine-containing transdermal dosage form for a second dosing period, wherein the second dosage form comprises the same or a greater dosage of buprenorphine than the first dosage form; and (c) administering to the patient a third buprenorphine-containing transdermal dosage form for a third dosing period, wherein the third dosage form comprises a greater dosage of buprenorphine than the second dosage form. Thus, for example, the first transdermal dosage form in the series may be worn by the patient for three consecutive days. Upon removal, the second dosage form may then be worn by the patient for another three consecutive days, and thereafter, the third dosage form may be worn by the patient for another seven days. In a preferred embodiment, the total treatment period of the dosing regimen is six days until the desired dose, i.e., the third dose level, is attained. This dose can then be maintained indefinitely. If an increase in dosage is required, then the dosage may be increased at an appropriate interval, e.g., every three days.

In a specific embodiment, the first dosage form comprises up to 5 mg buprenorphine, the first dosing period is at least about 2 days; the second dosage form comprises up to 10 mg buprenorphine, the second dosing period is at least about 3 days; the third dosage form comprises up to 20 mg buprenorphine, and the third dosing period is at least about 2 days. In another specific embodiment, the first and second dosing periods are about 7 days each.

In another embodiment, subsequent dosages may be administered. For example, if the target level for treatment for depression is attained with the third dosing period, the third dosage form can be continually administered for an indefinite period of time, changing patches with a frequency extending from about every 2 days or 10 days, or weekly. If needed in order to further alleviate depression and/or pain, subsequent dosage forms can be used incrementally starting with 30 mg buprenorphine and 40 mg buprenorphine load.

The dosage of buprenorphine may vary according to a variety of factors such as underlying disease state, the individual's condition, weight, sex and age and the mode of administration. The dosage regimen is selected in accordance with a variety of factors including type, species, age, weight, sex and medical condition of the patient; the severity of the condition to be treated; the route of administration; the renal and hepatic function of the patient; and the particular compound thereof employed. A physician of ordinary skill can readily determine and prescribe the effective amount of the drug required to prevent, counter or arrest the progress of the depression. Optimal precision in achieving concentrations of buprenorphine within the range that yields efficacy without toxicity requires a regimen based on the kinetics of buprenorphine's availability to target sites. This involves a consideration of the absorption, distribution, metabolism, and excretion of buprenorphine.

Transdermal patches can be placed, for example, on the right upper arm/shoulder, left upper arm/shoulder, right anterior thorax (subclavicular), left anterior thorax (subclavicular), right lower anterior axillary line, left lower anterior axillary line, right upper back, left upper back, or at the mid-axillary line at the fifth intercostal space. Repeated doses may or may not be administered to the same location each time.

"Sequential" administration of a transdermal patch includes the situation where a first dosage form is removed before a second patch is administered, as well as staggered administration regimens. Thus, in one embodiment, a patient only carries one transdermal patch at a time. In another embodiment, the administration of two sequential patches is staggered, in which case the patient may carry two patches containing the same or different doses for a suitable time, *e.g.,* up to 1 day, before the first administered patch is removed.

In yet another embodiment, a patient is simultaneously administered two transdermal patches to reach a particular dosage level of buprenorphine, e.g., two BTDS 5 to achieve a total dosage level of 10 mg buprenorphine.

The dosage forms used in the method of the present invention may be administered alone or in combination with other active agents. For combination treatment with more than one active agent, where the active agents are in separate dosage formulations, the active agents can be administered concurrently, or they each can be administered at separately staggered times. The dosage amount may be adjusted when combined with other active agents as described above. On the other hand, unit dosage forms of these various active agents may be independently optimized and combined to achieve a synergistic result wherein the pathology or condition is reduced more than it would be if either active agent were used alone. For example, the compound, pharmaceutical composition, or unit dosage form of the present invention may be administered alone or in combination with other SSRIs, tricyclics or MAOIs at appropriate dosages defined by routine testing in order to obtain optimal activity while minimizing any potential toxicity.

### EXAMPLES

The following Example(s) are understood to be exemplary only, and do not limit the scope of the invention or the appended claims.

### Example 1: Effectiveness of BTDS To Alleviate Depression in Elderly

This Example describes a randomized, double blind, pilot evaluation of the effectiveness of BTDS versus placebo (with short acting opioid therapy present in both groups) on health outcomes associated with analgesic management of elderly. The objective of the study is to explore differential health outcome experience in residents with chronic pain receiving usual analgesic care or usual care plus BTDS, including whether transdermal buprenorphine alleviates depression. The study is a double-blind, randomized, parallel arm, placebo-controlled trial, using 50 patients in each group.

***Patient Group.*** Elderly residents in nursing home/assisted living environment with chronic, non-malignant pain of musculoskeletal origin are recruited. The patient has a standing order for 2-6 tablets/capsules per day Darvocet, Darvon, Tylenol #3, Tylox, Percocet, Percodan, Lortab, Ultram, Vicodin, Lorcet or Zydone, and has taken between 5 and 42 tablets/capsules in the 7 days prior to enrollment but not greater than the equivalent of 90 mg of oral morphine in any single day. The subject reports a resident rating of "average pain in the last 24 hours" >4 (0-10 scale) on 3 out of 7 days. The patient has a Modified Mini-Mental State Exam (3MS) score ≥60, and sufficient mental acuity to participate in the study and is able to answer study questions. The resident desires to have an improvement in pain.

***Dosing Procedures.*** The test medication is either buprenorphine transdermal delivery system containing 5, 10, or 20 mg buprenorphine (BTDS 5, 10, or 20; Purdue Pharma L.P.), or reference/placebo (BTDS 5, 10 or 20 without buprenorphine). Each patient is randomized to either BTDS or placebo.

Because these are elderly residents in supervised living, it may take the study staff one or more contacts to establish an adequate baseline assessment. During the screening period, residents must have taken between 5 and 42 units of short-acting opioids in the previous week (but not more than the equivalent of 90 mg of oral morphine in any single day) and rated their average pain in the last 24 hours as > 4 (0-10) on 3 out of 7 days to be enrolled in the study. Day 0 will be the day of randomization despite the number of contacts required to complete baseline evaluations.

Each resident will begin the study (Day 0). On day 0, after all baseline evaluations are completed, residents will receive either 5 mg BTDS or a matching placebo and pre-randomization usual care short-acting opioid therapy is continued. The TDS will be applied every 7 days throughout the study unless titration to a different dose is warranted. Patients who are currently taking other CNS depressants, including, benzodiazepines, sedatives, hypnotics, general anesthetics, other opioid analgesics, phenothiazines, centrally acting anti-emetics and alcohol, should be dosed with caution. Residents starting on BTDS should be monitored for respiratory depression, hypotension, and over-sedation.

Study therapy will be titrated over 18 days to acceptable pain control. A minimum of 72 hours (3 days) is required on each dose before upward titration may be considered. Starting on Day 3, if the resident's average pain in the last 24 hours is > 4 (0-10) and side effect experience is deemed to be tolerable, the resident will be titrated to either a 10 mg BTDS or matching placebo. Access to usual care short-acting opioid therapy continues throughout the study.

Beginning three days after titration from the 5 mg dose, residents on the 10 mg dose (or matching placebo) will be asked to rate their average pain in the last 24 hours (0-10). If the rating is > 4 and side effect experience tolerable the resident will be titrated to either a 20 mg BTDS or matching placebo. The investigator may choose a slower up-titration interval but all up-titration must be completed by Day 18. It is anticipated that residents will be titrated to achieve an average pain ≤4 (effective pain management) within the constraint of side effect experience. The resident should wear the new TDS until the next scheduled TDS application, when the usual application schedule should be resumed. Residents may titrate downward one level each week if needed to manage known opioid side effects. Residents may be on 5, 10, or 20 mg of BTDS or matching placebo at the end of the titration period.

At the end of the titration period or at any time the investigator judges the resident is experiencing effective pain management, the resident will continue to receive the dose of BTDS and the system will be replaced every 7 days by a member of the study staff. Placement of the TDS must be rotated. All residents continue to have their usual care short-acting opioid available for use upon request as transcribed in the medical chart and medication record.

***BTDS Application.*** The following application procedure is followed. Selected body sites must be relatively hairless and clean. If the designated site is too hairy, the hair must be clipped, NOT shaved; if cleansing is required, clean the site with clear water only. No alcohol, oils, lotions, or soaps may be used. Let the skin dry completely. Remove the TDS from the foil pouch. Tear open the pouch at the small cut, taking care not to rip the TDS inside. While holding the smaller part of the protective backing, remove the larger part of the backing and apply the TDS to the skin, beginning at the exposed edge. Then remove the smaller portion of the protective backing and complete the placement. Apply the TDS to one of the following sites: Right upper arm/shoulder, left upper arm/shoulder, right anterior thorax (subclavicular), left anterior thorax (subclavicular), right lower anterior axillary line, left lower anterior axillary line, right upper back, left upper back. Each subsequent TDS should be applied to one of the sites listed above that has not been used for any of the previous TDS applications. Once in place, press the TDS down with the palm of your hand for approximately 45 seconds. This ensures adhesion around the edges. Do not rub the TDS. Only one TDS may be worn at any time.

***Depression Evaluation.*** The Geriatric Depression Scale-SF, a 15-question geriatric depression scale (Sheikh et al., Clin Gerontol 1986;5:165-73), is administered at baseline and at Week 6 (between Days 40 and 42, on the same day as the Cognitive Function Tests, see below). The questions included are:

| | | |
|---|---|---|
| Are you basically satisfied with your life? | Yes | NO |
| Have you dropped many of your activities and interests? | YES | No |
| Do you feel your life is empty? | YES | No |
| Do you often get bored? | YES | No |
| Are you in good spirits most of the time? | Yes | NO |
| Are you afraid that something bad is going to happen to you? | YES | No |
| Do you feel happy most of the time? | Yes | NO |
| Do you often feel helpless? | YES | No |
| Do you prefer to stay at home, rather than going out and doing new things? | YES | No |
| Do you feel you have more problems with memory than most? | YES | No |
| Do you think it is wonderful to be alive? | Yes | NO |
| Do you feel pretty worthless the way you are now? | YES | No |
| Do you feel full of energy? | Yes | NO |
| Do you feel that your situation is hopeless? | YES | No |
| Do you think that most people are better off than you are? | YES | No |

1 point is given for every answer in capitals. A Single Question (SQ) test, a one question instrument to assesses depression status (Williams et al., Am J Med 1999;106:36-43), is also administered at baseline ("Have you felt sad or depressed much of the time in the past year?").

***Orthostatic Hypotension Evaluation.*** Orthostatic hypotension is defined as a drop in systolic blood pressure (BP) of 20 mm Hg or more and or a drop in diastolic BP of 10 mm Hg or more at one or three minutes after standing up from a supine position (Luukinen et al., Arch Int Med 1999;159;273-280; Kinney et al., AACN's clinical reference guide, Mosby, 4th ed., 1998, p. 285-7; Potter, Mosby, 4^{th} ed., 1997, p. 633; Kenny et al., Clin Geriatrics 2000;8:1-4).

***Periodic Evaluations.*** On three of every seven days, residents will be asked to evaluate the following: Average Pain Intensity in last 24 Hours (0 = no pain, 10 = Pain as bad as you can imagine); Quality of sleep last night (1 = excellent, 2 = good, 3 = fair, 4 = poor, 5 = very poor); Number of nighttime awakenings due to pain last night; Acceptability of analgesic therapy (1 = excellent, 2 = good, 3 = fair, 4 = poor, 5 = very poor); Bowel Status: The coordinator will document whether the resident had a bowel movement in the last 24 hours.

***Pain Management Evaluations.*** Spending time with residents on several occasions if necessary and understanding that residents may express "pain" as "discomfort" or "aching" or in other terms (Parmelee, In: Lawton MP, Teresi J (Eds.), Annual Review of Gerontology and Geriatrics, New York: Springer; 1994, p. 281-301) is important in pain evaluation. Several types of questions will be used to evaluate pain and pain burden so that the sponsor may identify those questions that are most sensitive to analgesic treatment effect and most understandable and meaningful to the residents for future studies. Identification of all site/s of pain and, if more than one site, the site with the worst pain using the Modified McGill Pain Map (Lichtenstein et al., J Gerontol 1998;53:M361-71). Questions about average pain and worst pain, and pain relief are taken from the modified Brief Pain Inventory (In: Vibbert S, Migdail KJ, Strickland D, Youngs MT, (Eds.), The 1995 medical outcomes and guidelines sourcebook, New York, NY, Faulkner & Gray, Inc., 1994, p. 269-270). Questions about pain interference with activities are from the Medical Outcomes Study Pain Evaluation (Stewart A, Ware J, (Eds.), Measuring functioning and well-being the medical outcomes study approach, Durham and London: Duke University Press; 1992).

***Cognitive Function Tests.*** Cognitive function will be evaluated at baseline (Day 0), Day 28, and at end of study (between Days 40 and 42) to assess whether there are changes in cognitive status associated with opioid therapy and pain levels. There are published reports of cognitive impairment associated with chronic pain particularly in the areas of attention, processing speed and psychomotor speed (Bellamy et al., J Rheumatol 1988;15:1833-40). Therefore, tests that assess attention, concentration, and processing speed are included.

The Randomized Digit Lists for Span Test (Appendix P) is included to measure resident's focused attention (Digits Forward) and working memory (Digits Backward). These tests are sensitive to changes in attention and concentration. Tests are also included to measure attention and sequencing (Trail Making Test Part A, or "TMT"). This is a widely recognized test that is sensitive to measuring changes in cognitive function. Alternate forms are available for both digits lists for span test and TMT, which will be used to minimize practice effects.

***Statistics.*** Statistical programming and analyses will be performed using SAS (SAS Institute, Cary, NC). Unless otherwise specified, all statistical tests will be performed as two-sided tests with a significance level of α = 0.05 for main effects and α = 0.10 for interactions.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

Patents, patent applications, publications, procedures, and the like are cited throughout this application, the disclosures of which are incorporated herein by reference in their entireties.

## Claims

1. A method of treating depression, which method comprises administering a transdermal dosage form comprising buprenorphine to a patient suffering from depression, thereby alleviating one or more symptoms of depression in the patient.

2. The method of claim 1, wherein the transdermal dosage form is selected from the group consisting of a transdermal dosage article and a transdermal dosage composition.

3. The method of claim 2, wherein the transdermal dosage article is a diffusion driven transdermal system.

4. The method of claim 2, wherein the transdermal dosage composition is selected from the group consisting of a topical gel, a lotion, an ointment, a transmucosal system, a transmucosal device, and an iontophoretic delivery system.

5. The method of claim 1, wherein the transdermal dosage form comprises from about 5 to about 40 mg buprenorphine.

6. The method of claim 1, wherein the symptoms are selected from the group consisting of persistent sad mood, loss of interest or pleasure in activities that were once enjoyed, significant change in appetite or body weight, difficulty sleeping, oversleeping, physical slowing, agitation, loss of energy, feelings of worthlessness, inappropriate guilt, difficulty thinking, difficulty concentrating, and recurrent thoughts of death or suicide.

7. The method of claim 1, wherein the depression is classified as refractory depression.

8. The method of claim 1, wherein the patient is an elderly patient.

9. The method of claim 1, comprising repeating the step of administering the transdermal dosage form at least once.

10. The method of claim 9, comprising repeating the step of administering the transdermal dosage form at least 6 times.

11. The method of claim 10, wherein the repeating step is conducted every 3-7 days.

12. A method of treating depression, which method comprises sequential administration of a first, a second, and a third transdermal dosage article comprising buprenorphine to a patient suffering from depression, wherein the third dosage article comprises a higher dosage of buprenorphine than the first and second dosage article thereby alleviating one or more symptoms of depression in the patient.

13. The method of claim 12, wherein the first dosage article comprises no more than 5 mg buprenorphine, the second dosage article comprises no more than 10 mg buprenorphine and is administered for a dosing period of three days, and the third dosage article comprises at least 20 mg buprenorphine and is administered for a dosing period of at least 2 days.

14. The method of claim 12, wherein the first dosage article comprises no more than 10 mg buprenorphine, the second dosage article comprises no more than 20 mg buprenorphine and is administered for three days, and the third dosage article comprises at least 30 mg buprenorphine and is administered for at least 2 days.

15. The method of claim 12, wherein the first dosage article comprises no more than 20 mg buprenorphine, the second dosage article comprises no more than 30 mg buprenorphine and is administered for three days, and the third dosage article comprises 40 mg buprenorphine and is administered for at least 2 days.

16. The method of claim 12, wherein the second dosage article is administered between 3 and 7 days after the first dosage article.

17. The method of claim 16, wherein the third dosage article is administered between 3 and 7 days after the second dosage article.

18. The method of claim 17, further comprising repeating the administration of the third dosage article at least once.

19. The method of claim 18, comprising repeating the administration of the third dosage article at least 4 times.

20. The method of claim 12, wherein the patient is an elderly patient.
